Europäisches Patentamt

European Patent Office

Office européen des brevets

(11) Veröffentlichungsnummer: **0 189 834**
**A2**

## (12) EUROPÄISCHE PATENTANMELDUNG

(21) Anmeldenummer: 86100830.8

(22) Anmeldetag: 22.01.86

(51) Int. Cl.⁴: **C 07 D 215/06**

(30) Priorität: 26.01.85 DE 3502690

(43) Veröffentlichungstag der Anmeldung:
06.08.86 Patentblatt 86/32

(84) Benannte Vertragsstaaten:
DE FR GB IT

(71) Anmelder: BASF Aktiengesellschaft
Carl-Bosch-Strasse 38
D-6700 Ludwigshafen(DE)

(72) Erfinder: Ruland, Alfred Dr.
Schriesheimer Strasse 11
D-6945 Hirschberg(DE)

(72) Erfinder: Reuther, Wolfgang, Dr.
Am Pferchelhang 16
D-6900 Heidelberg(DE)

(72) Erfinder: Schoeppl, Hubert, Dr.
Zinkgraefstrasse 58
D-6940 Weinheim(DE)

(72) Erfinder: Paulus, Gerhard, Dr.
Roonstrasse 1
D-6940 Weinheim(DE)

(72) Erfinder: Boehm, Walter, Dr.
Mittlerer Waldweg 11
D-6719 Kirchheim(DE)

(72) Erfinder: Rexroth, Erhard, Dr.
Sudetenstrasse 10
D-6806 Viernheim(DE)

(72) Erfinder: Queins, Hubertus, Dr.
Leininger Strasse 7
D-6701 Friedelsheim(DE)

(54) Verfahren zur Herstellung von oligomerem 2,2,4-Trimethyldihydrochinolin.

(57) Verfahren zur Herstellung von oligomerem 2,2,4-Trimethyldihydrochinolin, das 2 bis 10 Monomereneinheiten aufweist, die aus Anilin und Aceton aufgebaut sind, durch Umsetzung von Anilin und Aceton oder dessen Derivaten, indem man die Umsetzung in Gegenwart von 0,03 bis 0,1 Mol Bortrifluorid als Katalysator, bezogen auf 1 Mol Anilin, durchführt, dabei Aceton oder dessen Derivate dem Anilin bei einer Temperatur von 130 bis 160°C zusetzt, das bei der Umsetzung entstehende Wasser kontinuierlich aus dem Reaktionsgemisch entfernt und das Reaktionsgemisch anschließend bei einer Temperatur von 80 bis 110°C nachbehandelt.

EP 0 189 834 A2

0189834

## Verfahren zur Herstellung von oligomerem 2,2,4-Trimethyldihydrochinolin

Die vorliegende Erfindung betrifft ein neues Verfahren zur Herstellung von oligomerem 2,2,4-Trimethyldihydrochinolin, ausgehend von Anilin und Aceton oder dessen Derivaten, indem man die Umsetzung in Gegenwart von Bortrifluorid als Katalysator vornimmt, dabei Aceton oder dessen Derivate dem Anilin bei einer Temperatur von 130 bis $160^0$C zusetzt, das bei der Umsetzung entstehende Wasser kontinuierlich entfernt und das Reaktionsgemisch anschließend bei einer Temperatur von 80 bis $110^0$C nachbehandelt.

Es ist bekannt, durch Reaktion von Aceton oder von Acetonderivaten, wie Isopropenylalkylethern, Diacetonalkohol oder Mesityloxid und Anilin oder substituierten Anilinen monomeres 2,2,4-Trimethyldihydrochinolin oder dessen Derivate herzustellen.

Als Katalysatoren wurden hierzu verschiedene Produkte vorgeschlagen. Beispielsweise sind Säuren, wie p-Toluolsulfonsäure, Sulfanilsäure oder Bromwasserstoff, elementares Jod oder auch Eisenchlorid oder Aluminium chlorid beschrieben worden (FR-A-2 183 194, JP-A-88 363/1983).

Auch die Verwendung von Bortrifluorid für diesen Zweck ist bekannt. Beispielsweise werden in der US-A-3 899 492 Bortrifluorid-Ethanol und in der JP-A-11 967/1982 ein Bortrifluorid-Anilin-Komplex genannt.

Die Herstellung oligomerer oder polymerer 2,2,4-Trimethyldihydrochinoline erfolgt im allgemeinen ausgehend vom monomeren Chinolinderivat. Das Monomere muß dazu in einer separaten Stufe in Gegenwart von Salzsäure oder Aluminiumchlorid oligomerisiert bzw. polymerisiert werden (US-A-2 718 517, GB-A-1 010 247).

In einigen Verfahren werden oligomere 2,2,4-Trimethyldihydrochinoline auch direkt bei der Umsetzung der oben genannten Ausgangsprodukte (auf Basis Aceton und Anilin) erhalten. So wird in der JP-A-11 103/1973 ein Verfahren beschrieben, das in Gegenwart von konzentrierter Salzsäure als Katalysator abläuft. Nach dem Vorschlag der JP-A-14 516/1981 wird Anilin mit Aceton zunächst in Gegenwart von p-Toluolsulfonsäure umgesetzt, danach wird die Reaktion mit konzentrierter Salzsäure fortgeführt.

Neben der Tatsache, daß die als Katalysator dienende Salzsäure bekanntlich in hohem Maße korrosiv wirkt, weswegen in diesen Verfahren nur besonders ausgerüstete Apparaturen zur Anwendung gelangen können, weisen die so erhaltenen Reaktionsmischungen neben den gewünschten oligomeren Produkten jeweils noch große Mengen an monomerem 2,2,4-Trimethyldihydrochinolin oder auch an polymerem Rückstand auf.

Hoe/P

Aufgabe der vorliegenden Erfindung war es nun, ein Verfahren bereitzustellen, das es erlaubt, oligomeres 2,2,4-Trimethyldihydrochinolin in einfacher Weise und mit guten Ausbeuten zu synthetisieren.

Es wurde gefunden, daß die Herstellung von oligomerem 2,2,4-Trimethyldihydrochinolin, das 2 bis 10 Monomereneinheiten aufweist, die aus Anilin und Aceton aufgebaut sind, durch Umsetzung von Anilin und Aceton oder dessen Derivaten vorteilhaft gelingt, wenn man die Umsetzung in Gegenwart von 0,03 bis 0,1 Mol Bortrifluorid als Katalysator, bezogen auf 1 Mol Anilin, durchführt, dabei Aceton oder dessen Derivate dem Anilin bei einer Temperatur von 130 bis 160°C zusetzt, das bei der Umsetzung entstehende Wasser kontinuierlich aus dem Reaktionsgemisch entfernt und das Reaktionsgemisch anschließend bei einer Temperatur von 80 bis 110°C nachbehandelt.

Als Ausgangsprodukte für das erfindungsgemäße Verfahren dienen Anilin und Aceton oder dessen Derivate. Unter Acetonderivaten sind im vorliegenden Fall Diacetonalkohol, Mesityloxid oder die niederen Alkylether des Isopropenols zu verstehen. Die Verwendung von Aceton als Ausgangskomponente ist bevorzugt.

Das Molverhältnis Anilin : Aceton beträgt im erfindungsgemäßen Verfahren zweckmäßig 1:1,7 bis 1:2,4. Werden dimere Acetonderivate (Diacetonalkohol, Mesityloxid) eingesetzt, so ist dies beim genannten Molverhältnis in entsprechender Weise zu berücksichtigen.

Das mittels des erfindungsgemäßen Verfahrens hergestellte oligomere 2,2,4-Trimethyldihydrochinolin weist 2 bis 10, vorzugsweise 3 bis 6 Monomereneinheiten auf. Die Monomereneinheiten sind aus Anilin und Aceton aufgebaut, wobei in der Regel ein Molekül Anilin und zwei Moleküle Aceton für die Chinolinbildung benötigt werden.

Abhängig von dem bei der Umsetzung gewählten Molverhältnis Anilin : Aceton (oder Acetonderivate), d.h. je nach dem, ob man die Umsetzung mit einem Überschuß an Anilin oder mit einem Überschuß an Aceton, jeweils bezogen auf das stöchiometrische Molverhältnis Anilin : Aceton von 2:1, durchführt, gelangt man aber auch zu Oligomeren deren Monomerglieder zusätzliche Anilin- oder Acetoneinheiten aufweisen.

Als Katalysator dient Bortrifluorid. Es kann entweder in reiner Form oder als Bortrifluorid-Komplex, z.B. Bortrifluorid-Etherat, Bortrifluorid-Essigsäure oder Bortrifluorid-Phosphorsäure eingesetzt werden. Zweckmäßig verwendet man reines Bortrifluorid oder Bortrifluorid-Etherat, wobei die Anwendung von reinem gasförmigen Bortrifluorid besonders bevorzugt ist.

Bortrifluorid wird in einer Menge von 0,03 bis 0,1 Mol, vorzugsweise 0,04 bis 0,06 Mol, jeweils bezogen auf ein Mol Anilin, als Katalysator zugesetzt. In manchen Fällen können diese Werte auch noch über- oder unterschritten werden, wobei aber Katalysatorkonzentrationen, die kleiner sind als 0,02 Mol, bezogen auf ein Mol Anilin, keine wirtschaftlichen Ergebnisse mehr liefern.

Das erfindungsgemäße Verfahren kann in An- oder Abwesenheit von inerten organischen Lösungsmitteln durchgeführt werden. Vorzugsweise arbeitet man in Gegenwart eines Lösungsmittels, das gleichzeitig als Schleppmittel für das bei der Umsetzung entstehende Wasser und zur Abtrennung von Aceton dienen kann. Geeignete Lösungsmittel sind beispielsweise Toluol, Xylol, Halogenaromaten oder höhersiedende Petroletherfraktionen. Besonders günstig ist die Anwendung von Toluol und Xylol.

Man arbeitet üblicherweise bei atmosphärischem Druck, jedoch ist auch die Arbeitsweise unter erhöhtem Druck möglich.

Zur Durchführung des erfindungsgemäßen Verfahrens legt man zweckmäßig Anilin, gelöst in einem organischen Lösungsmittel, vor. Dieser Lösung wird Bortrifluorid zugesetzt, und anschließend wird das Reaktionsgemisch auf eine Temperatur von 130 bis 160°C erhitzt. Ist dieser Temperaturbereich erreicht, so gibt man Aceton (oder dessen Derivate) in dem Maße zu, daß die genannte Temperatur eingehalten wird. Der Zusatz von Anilin oder dessen Derivaten erstreckt sich über einen Zeitraum von etwa 2 bis 20 Stunden, vorzugsweise 6 bis 10 Stunden.

Das bei der Umsetzung entstehende Reaktionswasser wird dabei zusammen mit einem Teil des Lösungsmittels, des Acetons und des Anilins kontinuierlich aus dem Reaktionsgemisch abdestilliert. In einem Wasserabscheider wird von der wäßrigen Phase abgetrennt und die organische Lösungsmittel-Anilin-Aceton-Phase in das Reaktionsgemisch rückgeführt.

Für den Falll, daß man in Abwesenheit eines Lösungsmittels arbeitet, destilliert man kontinuierlich ein Gemisch aus Aceton und Wasser aus dem Reaktionsgemisch ab, wobei dieses Gemisch einer fraktionierenden Destillation unterworfen werden kann. Das dort gewonnene Aceton läßt sich ebenfalls in das Reaktionsgemisch rückführen.

Nach Beendigung der Acetonzufuhr, nach der sich im allgemeinen auch kein Reaktionswasser mehr abscheidet, wird das Reaktionsgemisch bei einer Temperatur von 80 bis 110°C nachbehandelt. Die Nachbehandlung wird in einem Zeitraum von 4 bis 8 Stunden, vorzugsweise 5 bis 6 Stunden vorgenommen.

Danach kühlt man das Reaktionsgemisch auf eine Temperatur von ca. 50°C ab und gibt gegebenenfalls weiteres Lösungsmittel zu.

Man wäscht intensiv mit verdünnter Natronlauge, trennt die organische Phase ab und unterwirft sie einer Destillation unter vermindertem Druck. Dabei destillieren das Lösungsmittel, nicht umgesetztes Anilin sowie als Nebenprodukt entstandenes monomeres 2,2,4-Trimethyldihydrochinolin ab. Als Rückstand bleibt das Zielprodukt, nämlich oligomeres 2,2,4-Trimethyldihydrochinolin.

Mittels des erfindungsgemäßen Verfahrens gelingt die Herstellung des Zielprodukts in einfacher Weise. Es fällt dabei in hoher Ausbeute (ca. 85 bis 95 Mol.%, bezogen auf eingesetztes Anilin) und hoher Reinheit (über 95 %) an, und die Bildung des monomeren Nebenproduktes bleibt auf geringe Mengen beschränkt (an 5 bis 15 Mol.%, bezogen auf eingesetztes Anilin).

Oligomeres 2,2,4-Trimethyldihydrochinolin dient als Alterungsschutzmittel für Kautschukvulkanisate.

Die nachfolgenden Beispiele sollen die Erfindung näher erläutern.

Beispiel 1

Ansatz:    930 g Anilin      (10     Mol)
           1276 g Aceton      (22     Mol)
            28 g $BF_3$-Gas   ( 0,41 Mol)
           600 ml Xylol

Anilin, 200 ml Xylol und $BF_3$-Gas wurden vorgelegt, dann bei einer Reaktionstemperatur von 140°C das Aceton so zudosiert, daß die Temperatur nicht unter 135°C abfiel. Gleichzeitig wurde Kondensationswasser und überschüssiges Aceton abdestilliert. Das Aceton wurde in einem Abscheider mit Hilfe des Xylols vom Wasser abgetrennt und in das Reaktionsgefäß zurückgeführt. Nach Ende der Acetonzufuhr, die 4 Stunden in Anspruch nahm, wurde 8 Stunden bei 100°C gerührt, anschließend mit 400 ml Xylol verdünnt und danach mit 10 %iger NaOH neutralisiert. Nach dem Abtrennen der Wasserphase wurde unter vermindertem Druck (ca. 20 mbar) soweit ausdestilliert, bis ein Erweichungspunkt (Ep) des Rückstands von 92 $\pm$ 5°C erreicht war.

Ausbeute oligomeres Chinolin = 1600 g = 93,5 % (bez. auf eingesetztes Anilin)

$$Ep = 90,3^0 C$$
$$\text{basischer Stickstoff} = 7,8 \%$$
$$\text{Alkalität} = 560 \text{ mg } HClO_4/g$$

**Beispiel 2** (Vergleich)

Ansatz:  930 g Anilin    (10   Mol)
        1276 g Aceton    (22   Mol)
          28 g BF$_3$-Gas  ( 0,41 Mol)
         600 ml Xylol

Anilin, 200 ml Xylol und BF$_3$-Gas wurden vorgelegt, dann bei einer Reaktionstemperatur von 140$^0$C das Aceton so zudosiert, daß die Temperatur nicht unter 135$^0$C abfiel. Gleichzeitig wurde Kondensationswasser und überschüssiges Aceton abdestilliert. Das Aceton wurde in einem Abscheider mit Hilfe des Xylols vom Wasser abgetrennt und in das Reaktionsgefäß zurückgeführt. Nach Ende der Acetonzufuhr wurde nur 2 Stunden bei 100$^0$C gerührt, anschließend mit 400 ml Xylol verdünnt und danach mit 10 %iger NaOH neutralisiert. Nach dem Abtrennen der Wasserphase wurde unter vermindertem Druck (ca. 20 mbar) soweit ausdestilliert, bis ein Erweichungspunkt des Rückstands von 92 ± 5$^0$C erreicht war.

Ausbeute oligomeres Chinolin = 337 g = 19,7 % (bez. auf eingesetztes Anilin)

$$Ep = 92,9^0 C$$
$$\text{basischer Stickstoff} = 6,72 \%$$
$$\text{Alkalität} = 482,8 \text{ mg } HClO_4/g$$

**Beispiel 3**

Ansatz:  930 g Anilin    (10   Mol)
        1044 g Aceton    (18   Mol)
          34 g BF$_3$-Gas  ( 0,5 Mol)
         600 ml Xylol

Anilin, 200 ml Xylol und BF$_3$-Gas wurden vorgelegt, dann bei einer Reaktionstemperatur von 140$^0$C das Aceton so zudosiert, daß die Temperatur nicht unter 135$^0$C abfiel. Gleichzeitig wurde Kondensationswasser und überschüssiges Aceton abdestilliert. Das Aceton wurde in einem Abscheider mit Hilfe des Xylols vom Wasser abgetrennt und in das Reaktionsgefäß zurückgeführt. Nach Ende der Acetonzufuhr wurde 8 Stunden bei 100$^0$C gerührt, anschließend mit 400 ml Xylol verdünnt und danach mit 10 %iger NaOH neutralisiert. Nach dem Abtrennen der Wasserphase wurde unter vermindertem Druck (ca. 20 mbar) soweit ausdestilliert, bis ein Erweichungspunkt des Rückstands von $92 \pm 5^0$C erreicht war.

Ausbeute oligomeres Chinolin = 1305 g = 76,3 % (bez. auf eingesetztes Anilin)

$$Ep = 93,0^0C$$
basischer Stickstoff = 8,27 %
Alkalität = 593,4 mg $HClO_4$/g

Beispiel 4

Ansatz:    930 g Anilin      (10    Mol)
           930 g Aceton      (16    Mol)
            34 g $BF_3$-Gas   ( 0,5  Mol)
           600 ml Xylol

Anilin, 200 ml Xylol und $BF_3$-Gas wurden vorgelegt, dann bei einer Reaktionstemperatur von $140^0$C das Aceton so zudosiert, daß die Temperatur nicht unter $135^0$C abfiel. Gleichzeitig wurde Kondensationswasser und überschüssiges Aceton abdestilliert. Das Aceton wurde in einem Abscheider mit Hilfe des Xylols vom Wasser abgetrennt und in das Reaktionsgefäß zurückgeführt. Nach Ende der Acetonzufuhr wurde 8 Stunden bei $100^0$C gerührt, anschließend mit 400 ml Xylol verdünnt und danach mit 10 %iger NaOH neutralisiert. Nach dem Abtrennen der Wasserphase wurde unter vermindertem Druck (ca. 20 mbar) soweit ausdestilliert, bis ein Erweichungspunkt des Rückstands von $92 \pm 5^0$C erreicht war.

Ausbeute oligomeres Chinolin = 1098 g = 64,2 % (bez. auf eingesetztes Anilin)

$$Ep = 93,6^0C$$
basischer Stickstoff = 8,6 %
Alkalität = 617,2 mg $HClO_4$/g

Die Beispiele 3 und 4 zeigen, daß bei vorgegebenem Erweichungspunkt mit der Verminderung des Acetonanteils auch eine Ausbeuteminderung an oligomerem Chinolin einhergeht.

0189834

Patentansprüche

1. Verfahren zur Herstellung von oligomerem 2,2,4-Trimethyldihydrochinolin, das 2 bis 10 Monomereneinheiten aufweist, die aus Anilin und Aceton aufgebaut sind, durch Umsetzung von Anilin und Aceton oder dessen Derivaten, dadurch gekennzeichnet, daß man die Umsetzung in Gegenwart von 0,03 bis 0,1 Mol Bortrifluorid als Katalysator, bezogen auf 1 Mol Anilin, durchführt, dabei Aceton oder dessen Derivate dem Anilin bei einer Temperatur von 130 bis 160$^0$C zusetzt, das bei der Umsetzung entstehende Wasser kontinuierlich aus dem Reaktionsgemisch entfernt und das Reaktionsgemisch anschließend bei einer Temperatur von 80 bis 110$^0$C nachbehandelt.

2. Verfahren gemäß Anspruch 1, dadurch gekennzeichnet, daß man die Umsetzung mit Aceton durchführt.

3. Verfahren gemäß Anspruch 1, dadurch gekennzeichnet, daß man Anilin und Aceton in einem Molverhältnis von 1:1,7 bis 1:2,4 einsetzt.

4. Verfahren gemäß Anspruch 1, dadurch gekennzeichnet, daß man die Umsetzung in Gegenwart eines inerten organischen Lösungsmittels durchführt.

5. Verfahren gemäß Anspruch 1, dadurch gekennzeichnet, daß man Aceton dem Anilin in einem Zeitraum von 2 bis 20 Stunden zusetzt.

6. Verfahren gemäß Anspruch 1, dadurch gekennzeichnet, daß man die Nachbehandlung in einem Zeitraum von 4 bis 8 Stunden vornimmt.